# EUROPEAN PATENT APPLICATION

(11) **EP 4 230 139 A1**
(43) Date of publication of application: **23.08.2023**
(21) Application number: 22192076.2
(22) Date of filing: 25.08.2022
(51) Int. Cl.: A61B 5/347, A61B 5/00

(54) **MEDICAL DATA PROVIDING DEVICE, MEDICAL DATA PROVIDING METHOD AND COMPUTER PROGRAM**

(30) Priority: 21.02.2022 KR 20220022463
(71) Applicant: Atsens Co., Ltd., Seongnam-si Gyeonggi-do 13637 (KR)
(72) Inventor: CHA, Kab Mun, Seongnam-si, Gyeonggi-do, Republic of Korea (KR); KIM, Tae Youn, Seongnam-si, Gyeonggi-do, Republic of Korea (KR); JEONG, Jong Ook, Seongnam-si, Gyeonggi-do, Republic of Korea (KR)
(74) Representative: Locas, Davide

(57) **Abstract**

A medical data providing device includes a memory, a communication unit, and a processor, wherein the processor is configured to read a biosignal stored in the memory, divide the biosignal at certain time intervals, convert a signal segment into the frequency , determines whether the signal segment is an analysis necessary section or an analysis unnecessary section based on a frequency component of the signal segment, further analyzes the signal segment of the analysis necessary section to generate data including an analysis result, and transmits data including the analysis result to a certain device.

## Description

### CROSS-REFERENCE TO RELATED APPLICATION

This application is based on and claims priority under 35 U.S.C. §119 to Korean Patent Application No. 10-2022-0022463, filed on February 21, 2022, in the Korean Intellectual Property Office, the disclosure of which is incorporated by reference herein in its entirety.

### BACKGROUND

### 1. Field

One or more embodiments relate to a medical data providing device, a medical data providing method, and a computer program, and more particularly, to converting a biosignal into the frequency domain and determining whether additional analysis of the biosignal is required based on a frequency component of the biosignal.

### 2. Description of the Related Art

When the heart muscle contracts and relaxes, a potential difference is generated by electrical depolarization and repolarization, and thus, it is possible to obtain an electrocardiogram by attaching electrodes to the skin surface. The electrocardiogram has a magnitude of several tens of µV to several mV and a frequency band of less than 100 Hz.

In recent years, electrocardiogram (ECG) signals have been measured using a patch-type ECG measurement device. The patch-type ECG measurement device records ECG signals continuously for a long period of time (e.g., 7 to 14 days) while the patient has a normal daily life. Size of the recorded ECG signals is very large, and it takes a lot of time to analyze all of the recorded ECG signals.

In addition, an ECG signal includes a section for which analysis is unnecessary due to noise at the time of measurement. Accordingly, it is necessary to select sections requiring analysis of the ECG signal measured for a long time.

Information disclosed in this background section was already known to the inventors before achieving the inventive concept or is technical information obtained in the process of achieving the inventive concept. Therefore, it may include information that does not form the prior art that is already known to the public.

### SUMMARY

One or more embodiments include a medical data providing device, a medical data providing method, and a computer program.

Additional aspects will be set forth in part in the description which follows and, in part, will be apparent from the description, or may be learned by practice of the presented embodiments of the disclosure.

According to one or more embodiments, a medical data providing device includes a memory, a communication unit, and a processor, wherein the processor reads a biosignal stored in the memory, divides the biosignal at certain time intervals, converts a divided first signal segment into the frequency domain, determines whether the first signal segment is an analysis necessary section or an analysis unnecessary section based on a frequency component of the first signal segment, further analyzes the first signal segment of the analysis necessary section to generate data including an analysis result, and transmits data including the analysis result to a certain device.

The processor may convert a first signal segment into frequency-domain data, extract one or more frequency values of one or more peaks included in the first signal segment, and set the first signal segment as an analysis unnecessary section when there are divisor or multiple frequencies of a largest peak frequency in the first signal segment.

The processor may convert the first signal segment into frequency-domain data, calculate one or more magnitudes of one or more peaks included in the signal segment, set the frequency value of the point of the peak of the highest value as a first frequency, determine whether a plurality of peaks having one or more frequencies of a multipul relation of the first frequency exist in the first signal segment, and the first signal segment is set as an analysis unnecessary section when there are a plurality of peaks in the first signal segment.

When the magnitude of data of one or more first peaks included in the first signal segment is less than a preset reference peak value, the processor may set the first signal segment as an analysis unnecessary section.

When a frequency magnitude of one or more second peaks included in the first signal segment exceeds a preset reference frequency value, the processor may set the first signal segment as an analysis unnecessary section.

The processor may perform a Fourier transform or a fast Fourier transform with a certain sample frequency of the biosignal.

The processor may perform a Fourier transform with the number of samples corresponding to certain time intervals of the biosignal.

The processor may preprocess the signal segment before converting a signal segment of the biosignal into the frequency domain.

The processor may preprocess the signal segment of the biosignal using a zero padding method or a data interpolation method.

The processor may determine whether the first signal segment is an analysis unnecessary section or an analysis necessary section using an analysis model generated by machine learning.

The processor may determine a length of the time interval using the analysis model.

The processor may determine the reference peak value or the reference frequency value using the analysis model.

According to one or more embodiments, a medical data providing method includes: reading a biosignal and dividing the biosignal at certain time intervals by a medical data providing device; converting, by the medical data providing device, a divided first signal segment into the frequency domain; determining, by the medical data providing device, whether the first signal segment is an analysis necessary section or an analysis unnecessary section based on a frequency component of the first signal segment; and generating, by the medical data providing device, data including an analysis result by further analyzing the first signal segment of the analysis necessary section and transmitting the data including the analysis result to a certain device.

According to one or more embodiments, a computer program is stored in a medium to execute any one of medical data providing methods according to an embodiment using a computer.

In addition to this, another method and another system for implementing the disclosure, and a non-transitory computer-readable recording medium for recording a computer program for executing the method are further provided.

Additional aspects will be set forth in part in the description which follows and, in part, will be apparent from the description, or may be learned by practice of the presented embodiments.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a view of a medical network system including a server and a user terminal according to an embodiment;
FIG. 2 is a view for explaining a detailed configuration of a medical data providing device according to embodiments; FIG. 3 is a block diagram of a medical data providing unit;
FIG. 4 is a view of a biosignal measurement system obtaining a biosignal according to embodiments;
FIG. 5 is a block diagram of biosignal measurement devices;
FIG. 6 is a flowchart illustrating a method of setting an analysis necessary section of a biosignal according to embodiments;
FIG. 7 is a flowchart illustrating a method of determining a signal segment including a harmonic component according to embodiments;
FIG. 8 is a flowchart illustrating a method of adjusting a time interval of a signal segment according to embodiments;
FIG. 9 is an exemplary view of a biosignal in an analysis unnecessary section;
FIG. 10A is an exemplary view of a biosignal having a low voltage value;
FIG. 10B is an example of a view of a biosignal measured in a state in which ECG sensing electrodes are not well attached to the skin;
FIG. 10C is an example of a view of a biosignal including noise having a high frequency; and
FIG. 11 is an example of a view of a biosignal including motion noise.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Reference will now be made in detail to embodiments, examples of which are illustrated in the accompanying drawings.

Since the disclosure may have diverse modified embodiments, preferred embodiments are illustrated in the drawings and are described in the detailed description. An effect and a characteristic of the disclosure, and a method of accomplishing these will be apparent when referring to embodiments described with reference to the drawings. The disclosure may, however, be embodied in many different forms and should not be construed as limited to the embodiments set forth herein.

Reference will now be made in detail to embodiments, examples of which are illustrated in the accompanying drawings, wherein like reference numerals refer to like elements throughout, and repeated description thereof will be omitted.

In this specification, terms such as "learning" are not intended to refer to mental operations such as human educational activities, but are interpreted as terms referring to performing neural network computing or machine learning through procedural computing. "Learning" refers to the study of computer algorithms that are automatically improved through experience.

It will be understood that although the terms "first," "second," etc. may be used herein to describe various elements, these elements should not be limited by these terms.

An expression used in the singular encompasses the expression of the plural, unless it has a clearly different meaning in the context.

It will be further understood that the terms "comprises" and/or "comprising" used herein specify the presence of stated features or elements, but do not preclude the presence or addition of one or more other features or elements.

In the following embodiments, a module may include a unit implemented in hardware, software, or firmware and may be used interchangeably with terms such as logic, logic blocks, components, or circuits. The module may be an integrally constructed component or a minimum unit or part of the component that performs one or more functions. For example, according to an embodiment, the module may be implemented in the form of an application-specific integrated circuit (ASIC).

Sizes of elements in the drawings may be exaggerated for convenience of description. In other words, since sizes and thicknesses of components in the drawings are arbitrarily illustrated for convenience of description, the following embodiments are not limited thereto.

When a certain embodiment may be implemented differently, a specific process order may be performed differently from the described order. For example, two consecutively described processes may be performed substantially at the same time or performed in an order opposite to the described order.

Hereinafter, a biosignal is a signal related to a living body, and may include a biosignal such as body temperature, pulse, electrocardiogram, brain wave, respiration rate, steps, stress, hormone, exercise amount, calories burned, body fat, body water, blood sugar value, and blood pressure.

Hereinafter, the biosignal may be an analog signal or a digital signal of a measured biosignal.

Hereinafter, a biosignal, which is a digital signal, refers to numerically processing a signal for modifying or improving an information signal in a desired direction.

Hereinafter, an electrocardiogram signal measures and diagnoses an abnormal heart rhythm, and may measure an abnormal rhythm due to damage in a nerve conductive tissue that transmits an electrical signal.

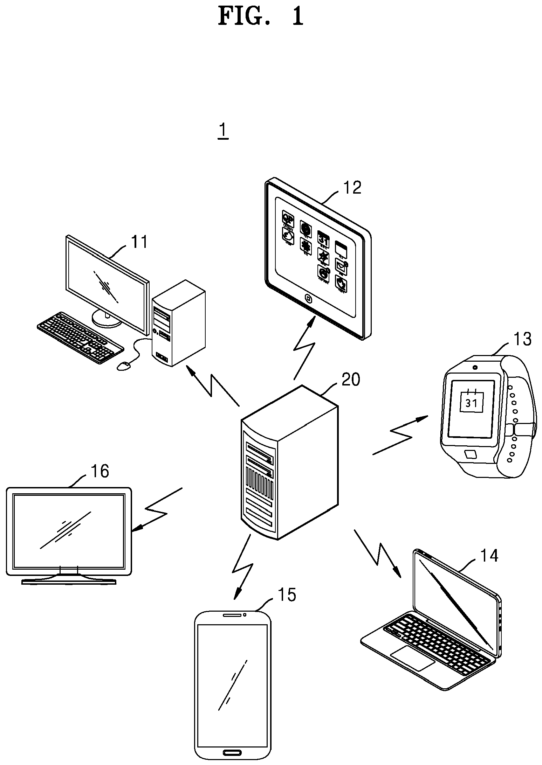

FIG. 1 shows a medical network system including a server and a user terminal according to an embodiment.

A medical network system 1 of the disclosure may include a server 20 and at least one of user terminals 11 to 16. The server 20 may provide various online activities through a network. The server 20 may simultaneously provide online activities to at least one of the user terminals 11 to 16.

According to an embodiment, the server 20 may include a single server, a group of servers, a cloud server, and the like, and is not limited to the above example. The server 20 provides various medical online activities and may include a database for storing data for network activities. In addition, the server 20 may include a payment server for generating and processing signal processing calculations or payment events. As described above, the server 20 may be a medical data providing device.

According to an embodiment, a network refers to connection established (or formed) using all communication methods, and may refer to a communication network connected through all communication methods for transmitting and receiving data between a terminal and a terminal or between a terminal and a server.

All communication methods may include communication through a certain communication standard, a certain frequency band, a certain protocol, or a certain channel. For example, all communication methods may include communication methods such as Bluetooth, BLE, Wi-Fi, Zigbee, 3G, LTE, and ultrasound, and may include short-range communication, long-range communication, wireless communication, and wired communication. However, all communication methods are not limited to the above examples.

According to an embodiment, a short-range communication method may refer to a communication method in which communication is possible only when a device (terminal or server) performing communication is within a certain range, and may include, for example, Bluetooth, NFC, and the like. A long-range communication method may refer to a communication method in which a device performing communication can communicate regardless of a distance. For example, the long-range communication method may refer to a method in which two devices performing communication through a repeater such as an AP may communicate even when they are separated from each other by a certain distance or more, and may include a communication method using a cellular network (3G or LTE) such as SMS or telephone. However, the long-range communication method is not limited to the above examples. The meaning of receiving medical online activity using a network may include the meaning that communication between a server and a terminal may be performed through all communication methods.

Throughout the specification, at least one of the user terminals 11 to 16 may include, but is not limited to, various electronic devices such as personal digital assistants (PDA), portable multimedia player (PMP), navigation, MP3 player, digital camera, refrigerator, washing machine, vacuum cleaner, and the like, in addition to the personal computer 11, the tablet PC 12, the mobile phone 13, the laptop computer 14, the smartphone 15, and the TV 16. As described above, at least one of the user terminals 11 to 16 may be a medical data providing device.

According to an embodiment, online activity may include, but is not limited to, a data processing service, a data analysis service, a data distribution service, a data transaction service, and the like.

According to an embodiment, the server 20 may determine whether analysis of a biosignal is necessary. The server 20 may divide the biosignal into signal segments and determine whether analysis of a signal segment is necessary. The server 20 may convert the signal segment into a frequency domain to determine whether analysis of the signal segment is necessary. The server 20 may upload a measured biosignal from a memory of a measurement device. The server 20 may convert the biosignal by a method such as a fast Fourier transform, a wavelet transform, or a Hilbert transform, but is not limited thereto and may convert the biosignal using various conversion methods.

In addition, according to an embodiment, the medical network system 1 may determine whether analysis of a biosignal is necessary. The medical network system 1 may divide a biosignal into signal segments and determine whether analysis of a signal segment is necessary. The medical network system 1 may convert the signal segment into a frequency domain to determine whether analysis of the signal segment is necessary. The medical network system 1 may upload a measured biosignal from a memory of the measurement device. The medical network system 1 may convert the biosignal by a method such as a fast Fourier transform, a wavelet transform, or a Hilbert transform, but is not limited thereto and may convert the biosignal using various conversion methods.

According to an embodiment, at least one of the user terminals 11 to 16 may determine whether analysis of a biosignal is necessary. At least one of the user terminals 11 to 16 may divide a biosignal into signal segments and determine whether analysis of a signal segment is necessary. At least one of the user terminals 11 to 16 may convert the signal segment into the frequency domain to determine whether analysis of the signal segment is necessary. At least one of the user terminals 11 to 16 may convert the biosignal by a method such as a fast Fourier transform, a wavelet transform, or a Hilbert transform, but is not limited thereto and may convert the biosignal using various conversion methods.

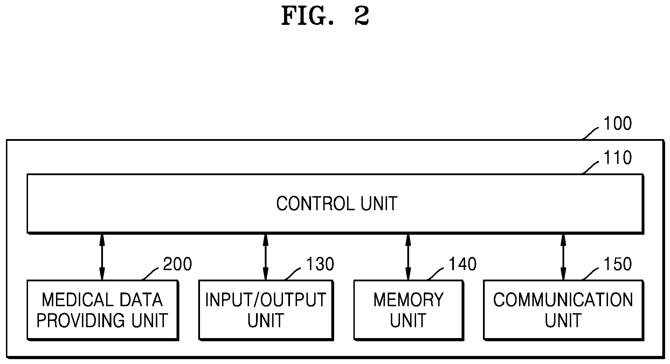

FIG. 2 is a view for explaining a detailed configuration of a medical data providing device according to embodiments.

As shown in FIG. 2, a medical data providing device 100 according to some embodiments may include a control unit 110, an input/output unit 130, a memory unit 140, a communication unit 150, and a medical data providing unit 200. However, not all of the components shown in FIG. 2 are essential components of the medical data providing device 100. That is, the medical data providing device 100 may include more or less components than the components shown in FIG. 2. The medical data providing device 100 may be a user terminal, a server, a medical data network system, or a separate device.

The control unit 110 generally controls all operations of the medical data providing device 100. For example, by executing a program stored in the medical data providing device 100, the control unit 110 may control all components included in the medical data providing device 100.

According to an embodiment, the control unit 110 may determine whether analysis of a biosignal is necessary. The control unit 110 may divide the biosignal into signal segments and determine whether analysis of a signal segment is necessary. The control unit 110 may convert the signal segment into a frequency domain to determine whether analysis of the signal segment is necessary. The control unit 110 may download a measured biosignal from a memory of a measurement device. The control unit 110 may convert the biosignal by a method such as a fast Fourier transform, a wavelet transform, or a Hilbert transform, but is not limited thereto and may convert the biosignal using various conversion methods.

The control unit 110 is a configuration for controlling the medical data providing device 100. In more detail, the control unit 110 controls all operations of the medical data providing device 100 by using various programs stored in the memory unit 140 of the medical data providing device 100. For example, the control unit 110 may include a central processing unit (CPU), random access memory (RAM), a read-only component (ROM), and a system bus. The ROM is a configuration in which an instruction set for system boot is stored, and the CPU copies an operating system stored in the medical data providing device 100 into the RAM according to an instruction stored in the ROM, and executes an O/S to boot a system. When system boot is complete, the CPU may perform various operations by copying various stored applications to the RAM and executing them. Although it has been described above that the medical data providing device 100 includes only one CPU, the medical data providing device 100 may be implemented with a plurality of CPUs (or a digital signal processor (DSP), a system on chip (SoC), etc.).

According to an embodiment, the control unit 110 may be implemented as a DSP processing a digital signal, a microprocessor, or a time controller (TCON). However, the control unit 110 is not limited thereto, and may include one or more of a CPU, a micro controller unit (MCU), a micro processing unit (MPU), a controller, an application processor (AP), and a communication processor (CP), or may be defined in a corresponding term. In addition, the control unit 110 may be implemented as a SoC or a large scale integration (LSI) in which a processing algorithm is embedded, or may be implemented in the form of a field programmable gate array (FPGA).

According to an embodiment, the input/output unit 130 may display an interface generated by the medical data providing device 100 by the memory unit 140. According to an embodiment, the input/output unit 130 may display a user interface for an input user input. The input/output unit 130 may output stored graphic data, visual data, auditory data, and vibration data by controlling the memory unit 140.

The input/output unit 130 may be implemented with various types of display panels. For example, a display panel may be implemented with various display technologies such as Liquid Crystal Display (LCD), Organic Light Emitting Diodes (OLED), Active-Matrix Organic Light-Emitting Diode (AM-OLED), Liquid Crystal on Silicon (LcoS), or Digital Light Processing (DLP). In addition, the input/output unit 130 may be coupled to at least one of a front area, a side area, and a rear area of the display panel in the form of a flexible display.

The input/output unit 130 may be implemented as a touch screen having a layer structure. The touch screen may have a function of detecting not only a display function but also a touch input position, a touched area, and even a touch input pressure, and may also have the ability to detect proximity touch as well as real-touch.

The input/output unit 130 may include a user interface for inputting various information to the medical data providing device 100. In addition, the input/output unit 130 may be located at a remote location.

According to an embodiment, the memory unit 140 may store a program for processing and controlling the control unit 110 and/or the medical data providing unit 200, and may also store data input/output to/from the medical data providing device 100. According to an embodiment, the memory unit 140 may store information about a user account or may store game-related information. The memory unit 140 may include a database storing the above information.

According to an embodiment, the memory unit 140 may include at least one type of storage medium from among memory of a flash memory type, a hard disk type, a multimedia card micro type or a card type (e.g., SD or XD memory), RAM, static random access memory (SRAM), ROM, electrically erasable programmable ROM (EEPROM), programmable ROM (PROM), magnetic memory, a magnetic disk, or an optical disk. In addition, according to an embodiment, programs stored in the memory unit 140 may be classified into a plurality of modules according to their functions. The programs may also be connected to a network in various types.

According to an embodiment, the communication unit 150 may communicate with an external device of the control unit 110. For example, the communication unit 150 may communicate with an external device such as a payment server or an authentication server under the control of the control unit 110. In addition, the communication unit 150 may obtain user information or a user input through communication with an external interface.

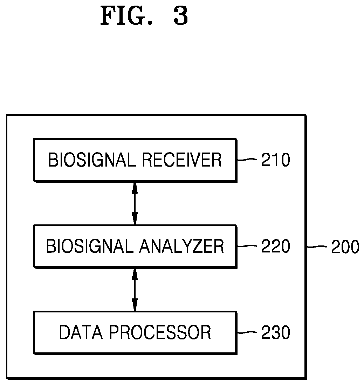

As shown in FIG. 3, the medical data providing unit 200 may include a biosignal receiver 210, a biosignal analyzer 220, and/or a data processor 230.

The biosignal receiver 210 may receive a biosignal. The biosignal receiver 210 may receive a biosignal from a database. The biosignal receiver 210 may receive a biosignal from a biosignal measuring device. This biosignal may be received from the communication unit 150, and this biosignal may be used for authentication and the like to protect personal information.

The biosignal analyzer 220 may divide a biosignal into a plurality of signal segments.

A biosignal may be divided into n signal segments at certain time intervals. Where n is a natural number. A time interval may be determined considering a sampling frequency of a signal and a conversion length in the frequency domain. For example, when a sampling frequency of a biosignal is 300 hz and a conversion length is 4096, the time interval may be determined as 4096/300 = 13.56 seconds. When a sampling frequency of a biosignal is 100 hz and a conversion length is 256, the time interval may be determined as 256/100 = 2.56 seconds. The time interval may be increased in proportion to the conversion length. When the time interval is too long, an overlapping portion of a normal section and an abnormal section may not be distinguished. When the conversion length is reduced, an error may occur in finding a frequency of a peak.

In another embodiment, when a heart rate value of a biosignal is changed within a time interval, the biosignal analyzer 220 may adjust inversely proportional to the time interval. In another embodiment, when a plurality of biosignals are included, the biosignal analyzer 220 may adjust a time interval for dividing a first biosignal considering a second biosignal. The time interval may be adjusted by comparing biosignals of a corresponding section.

The biosignal analyzer 220 may convert a signal segment of a biosignal into a frequency domain and divide the signal segment into an analysis necessary section and an analysis unnecessary section. The biosignal analyzer 220 may process the signal segment of the biosignal as an analysis unnecessary section or an analysis necessary section. The biosignal analyzer 220 may process the signal segment of the analysis necessary section in a different way. The biosignal analyzer 220 may process the signal segment of the analysis unnecessary section in a different way.

The biosignal analyzer 220 may convert a biosignal of a certain time interval into the frequency domain and extract one or more frequency valuesfor one or more peaks included in a signal segment of the converted biosignal. The biosignal analyzer 220 may set a first signal segment as an analysis unnecessary section when frequencies having a relationship of a factor and a multiple are included in a plurality of frequency valuesincluded in the first signal segment. A peak value may be an absolute value with respect to a complex-dimensional value.

In another embodiment, the biosignal analyzer 220 may set the first signal segment as an analysis unnecessary section when the first signal segment includes one or more harmonics. When a biosignal is an electrocardiogram signal, a biosignal of arrhythmia may not include clear harmonic components. A section that does not include a harmonic component may be determined as a signal segment of arrhythmia and set as a section requiring analysis. In an actually measured biosignal, a certain value (e.g., 90 %) or more may include a harmonic component. A signal segment that does not include a harmonic component may be a part. By extracting a signal segment of an analysis necessary section as in the embodiment, it is possible to reduce the amount of data for performing an additional classification or analysis process on a biosignal and reduce the analysis time, power, and memory capacity required for the additional classification or analysis process.

The biosignal analyzer 220 may convert a first signal segment into a frequency domain, extract one or more peaks included in the first signal segment. The biosignal analyzer 220 may determine whether frequencies having a multiple relationship with the smallest frequency value exist among the frequencies of the one or more peaks, and determine whether the frequency values of the peaks are greater than a preset reference frequency value. As a result of the determination, the biosignal analyzer 220 may set the first signal segment as an analysis unnecessary section. The biosignal analyzer 220 determines that the signal segment in which the respective frequency values corresponding to peaks are greater than a preset reference value is a normal section and does not further perform an additional analysis process.

The biosignal analyzer 220 may determine whether noise is included in a signal segment. A signal segment including noise is data unnecessary for analyzing user's heart abnormality, and may be determined as an analysis unnecessary section.

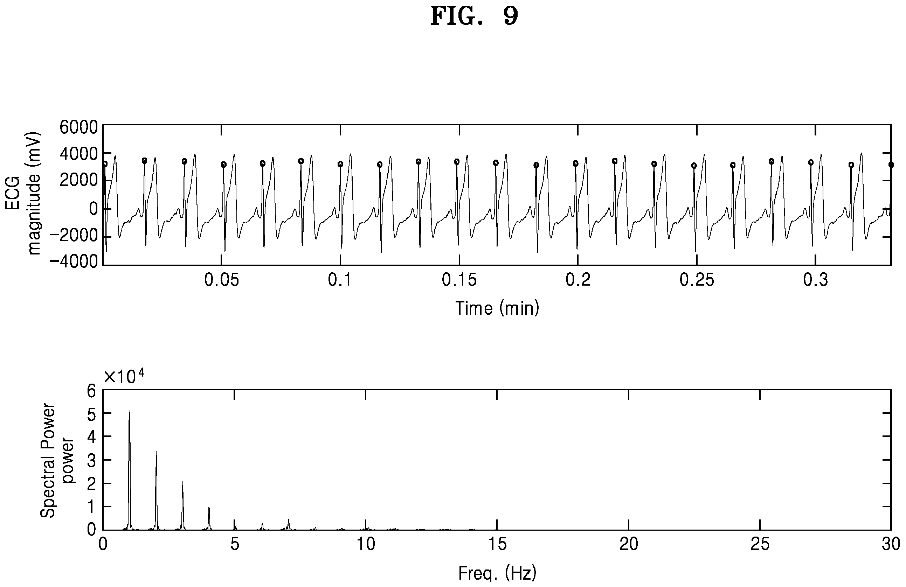

As shown in FIG. 9, a biosignal in a normal section, that is, in an analysis unnecessary section, has regular peaks. A regular peak is periodically generated in time and frequency, and data values of the peak may also have a constant value.

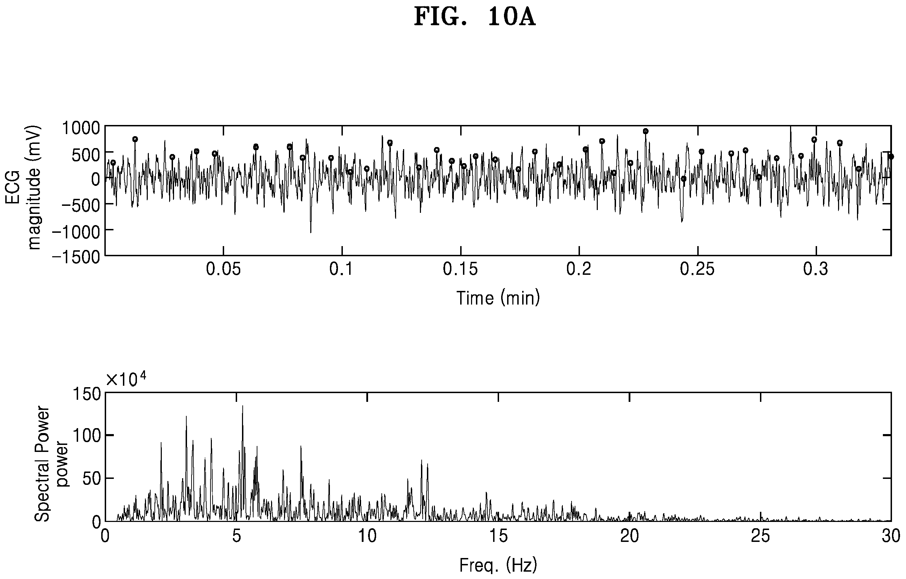

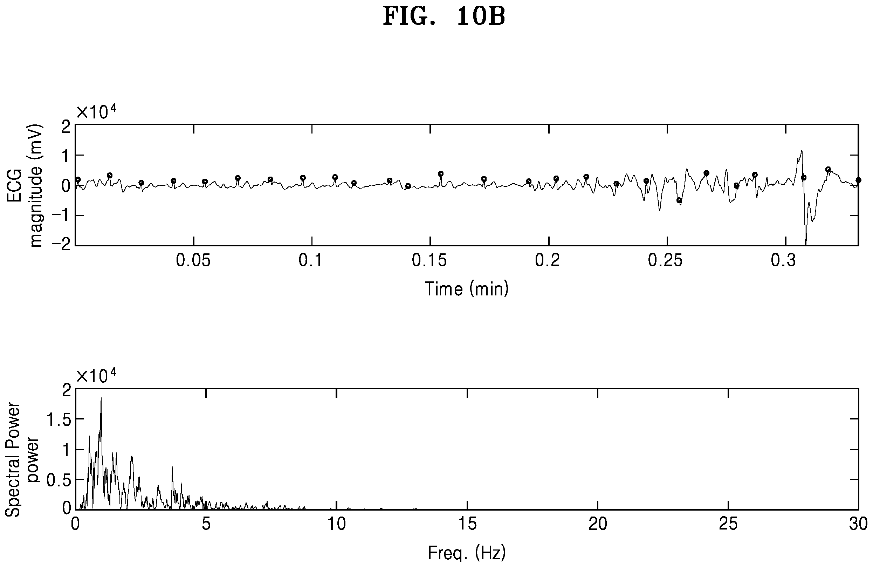

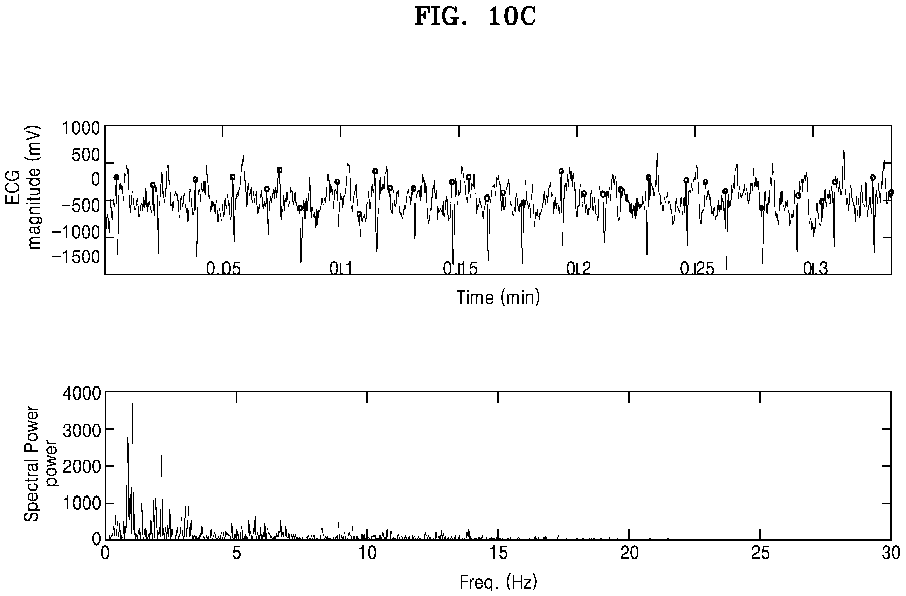

The biosignal may include noise as shown in FIGS. 10A, 10B, and 10C.

FIG. 10A is an exemplary view of a biosignal having a low voltage value. Peaks of the biosignal of FIG. 10A may have non-constant data values, and the generated time points may not be periodic. The biosignal of FIG. 10A may include irregular peaks. The biosignal including irregular peaks may be converted into the frequency domain to determine whether the biosignal is noise based on a peak value. In addition, it goes without saying that this procedure may be determined even before the frequency domain conversion.

FIG. 10B is an exemplary view of a biosignal measured in a state in which sensing electrode is not well attached to the skin. Although the biosignal of FIG. 10B includes peaks, the shape of the biosignal does not include a normal shape. It may be determined whether the biosignal that does not have a normal shape is noise based on a peak data value.

FIG. 10C is an exemplary view of a biosignal including noise having a high frequency. Because the biosignal of FIG. 10C also includes irregular peaks, it may be determined whether the biosignal is noise based on whether one or more frequency values exceeds a certain reference value. The noise may be determined whether data having a frequency exceeding a reference value is generated more than a predetmined number.

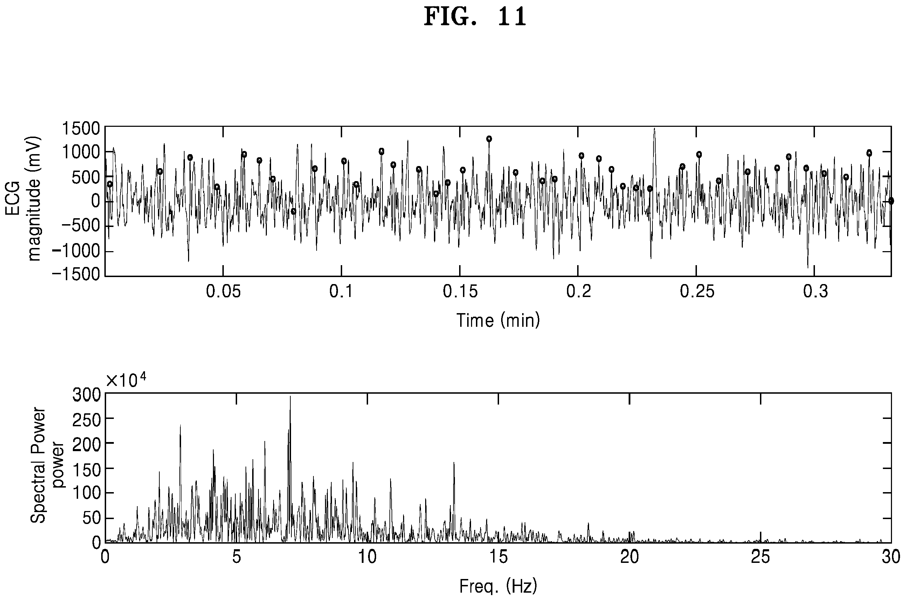

FIG. 11 is an exemplary view of a biosignal including motion noise.

The motion noise may be determined based on whether a frequency value of the biosignal exceeds a certain reference value. For example, when a frequency of peaks of a signal segment exceeds 5 hz, since the heart rate is determined to be 300 bpm or more, it may be determined that the signal segment includes motion noise.

When the magnitude (a data value) of a first peak included in a first signal segment is less than a preset reference peak value, the biosignal analyzer 220 may set the first signal segment as an analysis unnecessary section. Even if the first signal segment includes one or more harmonic components, when the magnitudes of the peaks are less than the reference peak value, the first signal segment may be set as an analysis unnecessary period. The reference peak value may be a value including a range of measurable data values. The reference peak value may be determined as measurable data values for each type of biosignal.

The biosignal analyzer 220 may extract a second frequency value from a second peak having the largest magnitude included in the first signal segment, and may set the first signal segment as an analysis unnecessary section when the second frequency value exceeds a preset reference frequency value. A signal segment having an unmeasurable level of frequency value may be determined to include noise, and may be set as an analysis unnecessary section. The reference frequency value may be a value including a range of frequency values measurable through a biosignal. The reference frequency value may be determined as measurable data values for each type of biosignal.

The biosignal analyzer 220 may execute Fourier transform a signal segment of a biosignal into a certain number of samples, and may generate a converted signal segment.

The biosignal analyzer 220 may preprocess the signal segment of the biosignal. The biosignal analyzer 220 may preprocess the signal segment of the biosignal using a zero padding method or a data interpolation method. In more detail, the biosignal analyzer 220 may set a portion of the signal segment to a zero value or a certain value. A section set to the zero value or the certain value may be a section satisfying a certain criterion. In another embodiment, the biosignal analyzer 220 may interpolate a biosignal or a signal segment by using measured values of a biosignal.

In another embodiment, a trained analysis model may be generated using a biosignal input to the biosignal analyzer 220 and a biosignal in an analysis unnecessary section or a biosignal in an analysis necessary section processed by the biosignal analyzer 220. The biosignal analyzer 220 may determine whether a signal segment of a biosignal is an analysis necessary section or an analysis unnecessary section using the analysis model.

The trained analysis model may be generated using a machine learning and a data mining. The machine learning predicts results based on known properties trained from training data, and data mining may discover unknown properties. Algorithms used to learn an analytic model may include supervised learning, unsupervised learning, semi-supervised learning, reinforcement learning, deep learning, and the like. An artificial neural network is a statistical learning algorithm inspired by a neural network in biology in machine learning and cognitive science, and refers to a model with problem-solving ability in which artificial neurons that form a network by synaptic bonding change the strength of synapses through learning. The analysis model may be generated by being trained by a teacher through the artificial neural network.

When one or more biosignals (e.g., an activity signal, PPG, phonocardiogram, or electrohysterogram) are simultaneously collected, an analysis unnecessary section may be determined by signals other than ECG.

The biosignal analyzer 220 may determine a length of an interval for dividing a biosignal by using the analysis model and analyze the biosignal with the length of the interval.

The biosignal analyzer 220 may determine a reference peak value or a reference frequency value using the analysis model.

The data processor 230 may store a signal segment of an analysis necessary section in a memory without storing a signal segment of an analysis unnecessary section in the memory. The data processor 230 may process a signal of the analysis unnecessary section and a signal of the analysis necessary section in response to a condition requested by a user. For example, the signal of the analysis unnecessary section and the signal of the analysis necessary section may be transmitted to a user who has requested the signal of the analysis unnecessary section and the signal of the analysis necessary section. To a user who has requested data about the signal of the analysis necessary section and the signal of the analysis necessary section, data about the signal of the analysis necessary section and the signal of the analysis necessary section may be transmitted.

In another embodiment, the data processor 230 may further perform an additional classification or analysis process on a signal segment of the analysis necessary section. The signal segment of the analysis necessary section may be stored together with data of a classified or analyzed result. The data processor 230 may not perform the additional classification or analysis process on a signal segment of the analysis unnecessary section.

In another embodiment, the data processor 230 may further calculate the heart rate of the signal segment of the analysis necessary section. The data processor 230 may not calculate the heart rate of the signal segment of the analysis unnecessary section.

The above example is only one example, and various additional classification or analysis processes may be further performed.

The medical data providing device 100 may detect the occurrence of a dangerous situation by analyzing a biosignal. The medical data providing device 100 may detect an analysis necessary section of a biosignal as a dangerous situation when the analysis necessary section is generated. The medical data providing device 100 may generate an analysis report about the analysis necessary section. The analysis report may include data about whether a normal beat is present, whether a blocked beat is present, whether a supraventricular ectopy beat, whether a ventricular ectopy beat, and the like. The analysis report may include, but is not limited to, information about the time of occurrence of an analysis necessary section, analysis information about the analysis necessary section (heart abnormality, heart disease-related information, and additional biometric valuessuch as pulse rate, body temperature, hormones, etc.), and the like, and may include various information about a biosignal analysis necessary section.

In another embodiment, the medical data providing device 100 may generate an analysis report by using an analysis result from another device. The medical data providing device 100 may transmit a biosignal of a plurality of analysis necessary sections to another device and receive analysis results for the analysis necessary sections. The medical data providing device 100 may generate an analysis report based on the analysis result. A module for generating an analysis report may be stored in advance.

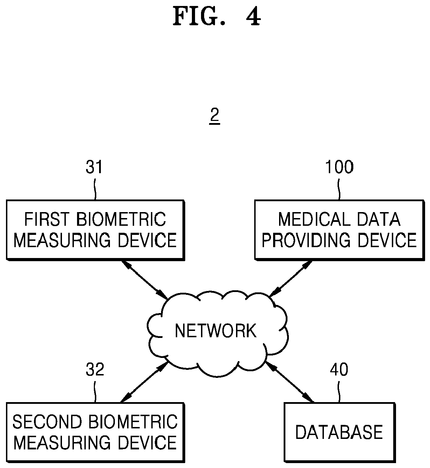

FIG. 4 is a view of a biosignal measurement system 2 obtaining a biosignal according to embodiments.

The biosignal measurement system 2 may include a medical data providing device 100, a first biometric measuring device 31, and a second biometric measuring device 32. Although the first biometric measuring device 31 and the second biometric device 32 are illustrated in FIG. 4, additional biometric devices may be further included. The first biometric measuring device 31 and the second biometric measuring device 32 may be a patch-type measuring device or a Holter-type measuring device.

The medical data providing device 100 may receive biosignals from the first biometric measuring device 31 and/or the second biometric measuring device 32 through a network. The network may include all communication methods such as communication through a certain communication standard, a certain frequency band, a certain protocol, or a certain channel. For example, all communication methods may include communication methods such as Bluetooth, BLE, Wi-Fi, Zigbee, 3G, LTE, and ultrasound, and may include short-range communication, long-range communication, wireless communication, and wired communication. However, the network is not limited to the above examples. According to an embodiment, a short-range communication method may refer to a communication method in which communication is possible only when a device (terminal or server) performing communication is within a certain range, and may include, for example, Bluetooth, NFC, and the like. A long-range communication method may refer to a communication method in which a device performing communication can communicate regardless of a distance. For example, the long-range communication method may refer to a method in which two devices performing communication through a repeater such as an AP may communicate even when they are separated from each other by a certain distance or more, and may include a communication method using a cellular network (3G or LTE) such as SMS or telephone.

The medical data providing device 100 may receive a biosignal from a device such as a database 40. The medical data providing device 100 may receive a biosignal, a biosignal processing signal, and the like from the first biometric measuring device 31, the second biometric measuring device 32, or the database 40. The medical data providing device 100 may process the received biosignal in response to the biosignal processing signal.

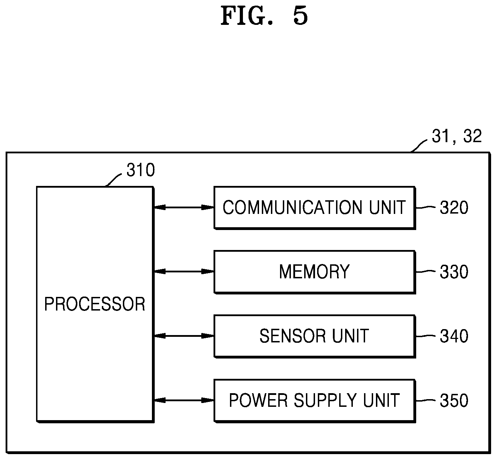

FIG. 5 is a block diagram of the biosignal measurement devices 31 and 32.

The biosignal measuring apparatuses 31 and 32 may include a processor 310, a communication unit 320, a memory 330, a sensor unit 340, and a power supply unit 350. The biosignal measuring apparatuses 31 and 32 may be mounted on an object non-invasively or invasively to measure electrocardiogram according to a heartbeat of the object. The biosignal measurement devices 31 and 32 may be implemented in a form that is attached to the skin or body of the object, but is not limited thereto and may be implemented in various ways. The object may be a human or animal, or a part of the body of a human or animal, such as a chest, but is not limited thereto, and any object may be considered if it can sense or measure an electrocardiogram. In addition, the electrocardiogram is a graph that records a potential change on a body surface according to a mechanical activity of a heartbeat, such as myocardial contraction/dilation, and the meaning of 'sensing an electrocardiogram' is the same as the meaning of 'sensing a potential' generated on a body surface according to a heartbeat of an object.

The processor 310 may generally control components such as the communication unit 320, the memory 330, the sensor unit 340, and the power supply unit 350. The processor 310 may store a biosignal measured by the sensor unit 340 in the memory 330. Data such as a biosignal stored in the memory 330 may be transmitted to an external device. The processor 310 may receive a control signal from the external device and process the data such as a biosignal according to the control signal. The processor 310 may process data such as a biosignal in response to a control signal stored in the memory 330. The processor 310 may generate a processed or analyzed biosignal in response to the control signal.

The processor 310 may divide the biosignal into a plurality of signal segments. The biosignal may be divided into n signal segments at certain time intervals. The processor 310 may change inversely proportionally the time intervals when a heart rate value of the biosignal is changed. When a plurality of biosignals are included, the processor 310 may adjust a time interval for dividing a first biosignal considering a second biosignal. The time interval may be adjusted by comparing biosignals of a corresponding section.

For example, when a sampling frequency of a biosignal is 300 hz and a conversion length is 4096, the time interval may be determined as 4096/300 = 13.56 seconds. When a sampling frequency of a biosignal is 100 hz and a conversion length is 256, the time interval may be determined as 256/100 = 2.56 seconds. The time interval may be increased in proportion to the conversion length. When the time interval is too long, an overlapping portion of a normal section and an abnormal section may not be distinguished. When the conversion length is reduced, an error may occur in finding a frequency of a peak.

The processor 310 may convert a signal segment of a biosignal into a frequency domain and divide the signal segment into an analysis necessary section and an analysis unnecessary section. The processor 310 may process the signal segment of the analysis necessary section in a different way. The processor 310 may process the signal segment of the analysis unnecessary section in a different way.

The processor 310 may convert a biosignal of a certain time interval into the frequency domain. The biosignal may be divided into a plurality of signal segments. The processor 310 may extract one or more frequency values for one or more peaks included in a signal segment. The processor 310 may set the first signal segment as an analysis unnecessary section when the first signal segment includes frequencies in a divisor relationship of frequencies in a multiple relationship. The peak value may be an absolute value with respect to a complex number value.

In another embodiment, the processor 310 may set the first signal segment as an analysis unnecessary section when the first signal segment includes one or more harmonics. When a biosignal is an electrocardiogram signal, a biosignal of arrhythmia may not include a harmonic component. A section that does not include a harmonic component may be determined as a signal segment of arrhythmia and set as a section requiring analysis. In an actually measured biosignal, a certain value (e.g., 90 %) or more may include a harmonic component. A signal segment that does not include a harmonic component may be a small portion of total electrocardiogram signal . By extracting a signal segment of an analysis necessary section as in the embodiment, it is possible to reduce the amount of data for performing an additional classification or analysis process on a biosignal and reduce the time, power, and memory capacity required for the additional classification or analysis process.

The processor 310 may convert the first signal segment into the frequency domain, extract one or more peaks included in the first signal segment. Here, the peak may refer to a point at which the slope value becomes 0, or a point at which the slope value changes from positive to negative and the slope value changes from negative to positive. The processor 310 may extract one or more points having a frequency in a multiple relation with the smallest frequency value from among the frequency values of the peaks. The processor 310 may calculate the number of peaks having a frequency greater than a preset reference frequency value. The processor 310 may set the signal segment as an analysis unnecessary section in consideration of the number of points having a frequency of a multiple relationship and the number of peaks having a frequency greater than the reference frequency value. The processor 310 determines that the signal segment in which magnitude values of the respective frequency corresponding to peaks are greater than a preset reference value is a normal section and does not further perform an additional analysis process.

When the magnitude (a data value) of a first peak included in a first signal segment is less than a preset reference peak value, the processor 310 may set the first signal segment as an analysis unnecessary section. Even if the first signal segment includes one or more harmonic components, when the magnitudes of the peaks are less than the reference peak value, the first signal segment may be set as an analysis unnecessary period.

The processor 310 may extract a second frequency value from a second peak having the largest magnitude included in the first signal segment, and may set the first signal segment as an analysis unnecessary section when the second frequency value exceeds a preset reference frequency value. A signal segment having an unmeasurable level of frequency value may be determined to include noise, and may be set as an analysis unnecessary section.

The processor 310 may Fourier convert a signal segment of a biosignal into a certain number of samples, and may generate a converted signal segment.

The processor 310 may preprocess the signal segment of the biosignal. The processor 310 may preprocess the signal segment of the biosignal using a zero padding method or a data interpolation method. In more detail, the processor 220 may set a portion of the signal segment to a zero value or a certain value. A section set to the zero value or the certain value may be a section satisfying a certain criterion. In another embodiment, the processor 310 may interpolate a biosignal or a signal segment by using measured values of a biosignal.

In another embodiment, a trained analysis model may be generated based on a biosignal input to the processor 310 and a biosignal in a processed analysis unnecessary section or a biosignal in an analysis necessary section. The processor 310 may determine whether a signal segment of a biosignal is an analysis necessary section or an analysis unnecessary section using the analysis model.

The processor 310 may determine a length of an interval for dividing a biosignal by using the analysis model and analyze the biosignal with the length of the interval.

The processor 310 may determine a reference peak value or a reference frequency value using the analysis model.

The processor 310 may store a signal segment of an analysis necessary section in a memory without storing a signal segment of an analysis unnecessary section in the memory. The processor 310 may process a signal of the analysis unnecessary section and a signal of the analysis necessary section in response to a condition requested by a user. For example, the signal of the analysis unnecessary section and the signal of the analysis necessary section may be transmitted to a user who has requested the signal of the analysis unnecessary section and the signal of the analysis necessary section. To a user who has requested data about the signal of the analysis necessary section and the signal of the analysis necessary section, data about the signal of the analysis necessary section and the signal of the analysis necessary section may be transmitted.

In another embodiment, the processor 310 may further perform an additional classification or analysis process on a signal segment of the analysis necessary section. The signal segment of the analysis necessary section may be stored together with data of a classified or analyzed result. The processor 310 may not perform the additional classification or analysis process on a signal segment of the analysis unnecessary section.

In another embodiment, the processor 310 may further calculate additional data such as a heart rate, body temperature, pulse, electrocardiogram, brain wave, respiration rate, steps, stress, hormone, exercise amount, calories burned, body fat, body weight, blood sugar value, blood pressure, whether there is a heart abnormality, or a related heart disease in the signal segment of the analysis necessary section. The processor 310 may not calculate additional data such as a heart rate, body temperature, pulse, electrocardiogram, brain wave, respiration rate, steps, stress, hormone, exercise amount, calories burned, body fat, body weight, blood sugar value, blood pressure, whether there is a heart abnormality, or a related heart disease in the signal segment of the analysis unnecessary section.

The above example is only one example, and various additional classification or analysis processes may be further performed. The processor 310 may detect the occurrence of a dangerous situation by analyzing a biosignal. The processor 310 may detect an analysis necessary section of a biosignal as a dangerous situation when the analysis necessary section is generated. The processor 310 may generate an analysis report of the analysis necessary section. The analysis report may include various data for the analysis necessary section. The analysis report may include a marker of a cardiac signals, an abnormal heartbeat, inadequate supply of blood and oxygen to the heart, an excessively thick heart muscle wall, information about a heart rate, and the like. The marker of the cardiac signal may be set to a normal beat (N) or at least one of a bundle branch block, a supraventricular ectopy beat (S, SVEB), and a ventricular ectopy beat (V, VEB).

For example, the processor 310 may include a CPU, RAM, ROM, and a system bus. The ROM is a configuration in which an instruction set for system boot is stored, and the CPU copies an operating system stored in the biosignal measurement devices 31 and 32 into the RAM according to an instruction stored in the ROM, and executes an O/S to boot a system. When booting is complete, the CPU may perform various operations by copying various stored applications to the RAM and executing them. Although it has been described above that the biosignal measurement devices 31 and 32 include only one CPU, the biosignal measurement devices 31 and 32 may be implemented with a plurality of CPUs (or a DSP, a separate function block, etc.).

The processor 310 may be implemented as a DSP, a microprocessor, or a TCON. However, the processor 310 is not limited thereto, and may include one or more of a CPU, an MCU, an MPU, a controller, an AP, a CP, and an advanced RISC machine (ARM) processor, or may be defined in a corresponding term. In addition, the processor 310 may be implemented as a SoC or a large scale integration (LSI) in which a processing algorithm is embedded, or may be implemented in the form of a field programmable gate array (FPGA).

The communication unit 320 may communicate with another device through a network.

The memory 330 may store a biosignal sensed by the sensor unit 340. The memory 330 may store a control signal for the biosignal and store a biosignal processed or analyzed in response to the control signal. The memory 330 may store one or more sensed biosignals. The memory 330 may store a sensed first biosignal and store a portion of a sensed second biosignal.

The sensor unit 340 detects a change in a physical or chemical phenomenon occurring in a person or an animal, and may sense values such as body temperature, pulse, electrocardiogram, brain wave, respiration rate, steps, stress, hormone, exercise amount, calories burned, body fat, body weight, blood sugar value, blood pressure, and the like.

The power supply unit 350 may supply power to each component of the biosignal measuring apparatuses 31 and 32.

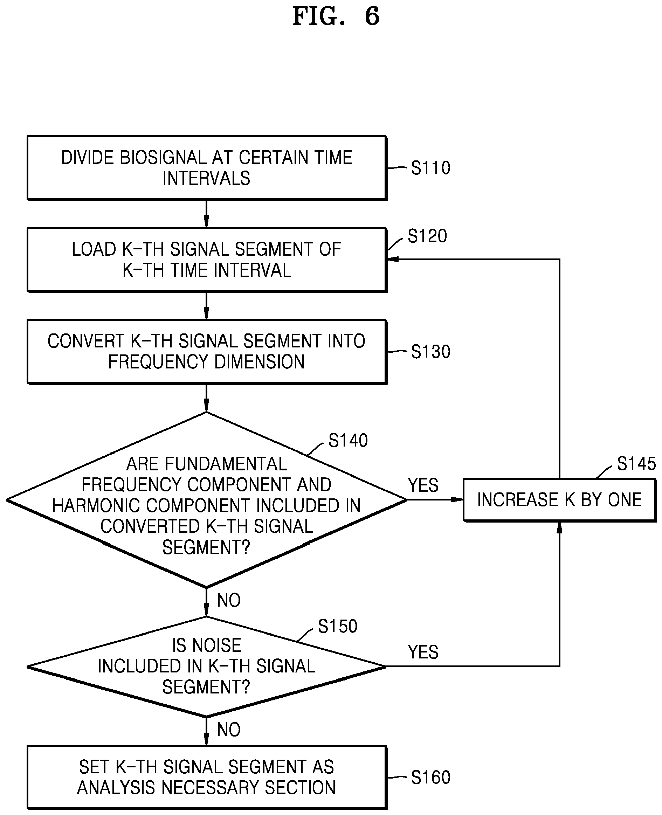

FIG. 6 is a flowchart illustrating a method of setting an analysis necessary section of a biosignal according to embodiments.

As shown in FIG. 6, in operation S110, the medical data providing device 100 may divide a biosignal at certain time intervals. One divided portion may be referred to as a signal segment. The time intervals for dividing the biosignal may be changeable. The medical data providing device 100 may preprocess the biosignal before converting the biosignal into the frequency domain.

In operation S120, the medical data providing device 100 may load a k^{th} signal segment of a k^{th} time interval.

In operation S130, the medical data providing device 100 may convert the k^{th} signal segment into the frequency domain. As a method of converting the k^{th} signal segment into the frequency domain, there may be one of a fast Fourier transform (FFT), a Fourier transform (FT), a wavelet transform, and a Hilbert transform. Converting into the frequency domain refers to converting data about time or space into frequency components. When a function for time is converted into the frequency domain, an amplitude at each frequency may represent a magnitude of a frequency component constituting the original function, and an angle may represent a phase offset from a basic sinusoid.

In operation S140, the medical data providing device 100 may determine whether a fundamental frequency component and a harmonic component are included in a converted k^{th} signal segment. The harmonic component refers to a peak having a frequency multiple of the fundamental frequency component. In operation S145, the medical data providing device 100 may move to operation S120 by setting the k^{th} signal segment including the fundamental frequency component and the harmonic component as an analysis unnecessary section and increasing k by one.

In operation S150, the medical data providing device 100 may determine whether noise is included in the k^{th} signal segment when the fundamental frequency component and the harmonic component are not included in the k^{th} signal segment. The medical data providing device 100 may determine that noise is included in the k^{th} signal segment when a frequency value of the k^{th} signal segment exceeds a reference frequency value or when the magnitude of a peak is less than a reference peak value.

In operation S160, when noise is not included, the medical data providing device 100 may set the k^{th} signal segment as an analysis necessary section.

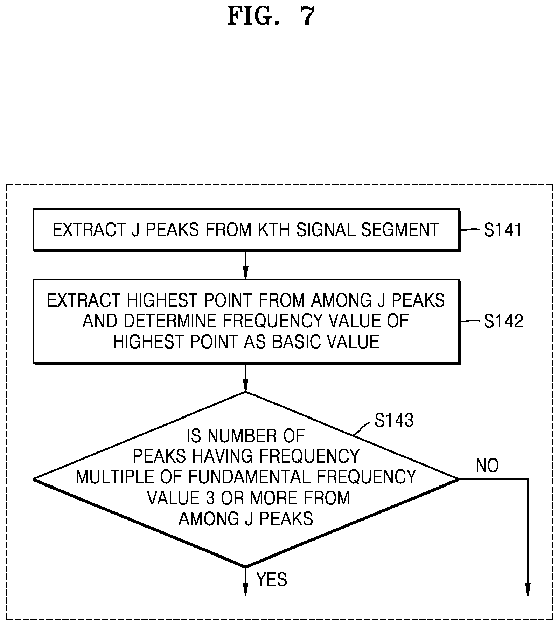

FIG. 7 is a flowchart illustrating a method of determining a signal segment including a harmonic component.

In operation S141, the medical data providing device 100 may extract j peaks from a k^{th} signal segment.

In operation S142, the medical data providing device 100 may extract a highest point from among the j peaks and determine a frequency value of the highest point as a basic value. The fundamental frequency value may be a complex value or an absolute value of the complex number. The highest value point refers to a point having the highest data value.

In operation S143, the medical data providing device 100 may determine whether the number of peaks having a frequency multiple of a fundamental frequency value is 3 or more from among the j peaks. When the number of peaks is three or more, the k^{th} signal segment may be determined as an analysis unnecessary section. Otherwise, the k^{th} signal segment may be determined as an analysis necessary section.

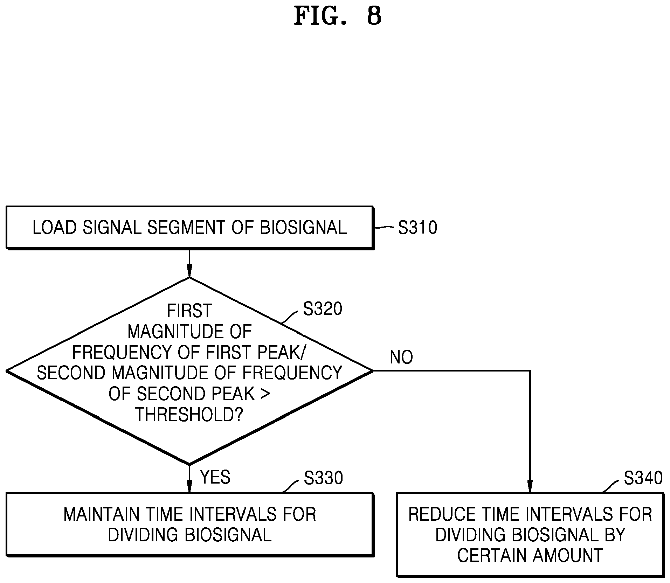

FIG. 8 is a flowchart illustrating a method of adjusting a time interval of a signal segment according to embodiments.

In operation S310, the medical data providing device 100 may load a signal segment of a biosignal.

In operation S320, the medical data providing device 100 may extract, from the signal segment, a first magnitude of a frequency of a first peak and a second magnitude of a frequency of a second peak, and may determine whether a ratio of the first magnitude to the second magnitude exceeds a threshold. The medical data providing device 100 may extract the first peak and the second peak from among peaks. The first peak may be a peak having a highest value, and the second peak may be a peak having a second highest value. The first magnitude may refer to a magnitude of a frequency of the peak having the highest value when the signal segment is converted into the frequency domain. The second magnitude may refer to a magnitude of a frequency of the peak having the second highest value.

When the ratio of the first magnitude to the second magnitude exceeds the threshold, it means that frequency values of the signal segment do not vary significantly. In operation S330, the medical data providing device 100 may maintain time intervals for dividing a biosignal when a ratio of a magnitude of a first frequency to a magnitude of a second frequency exceeds the threshold.

In operation S340, the medical data providing device 100 may reduce the time intervals for dividing the biosignal by a certain amount when the ratio of the first magnitude of the frequency of the first peak to the second magnitude of the frequency of the second peak is less than the threshold. When the ratio of the magnitude of the frequency of the first peak to the magnitude of the second frequency is less than the threshold, it may mean that the frequency values of the signal segment vary significantly. When the frequency values of the signal segment vary significantly, it may refer to a situation in which an object moves due to exercise or has an increased heart rate due to a certain cause.

The threshold is set to a certain value and may be changed because of machine learning.

Although the embodiments have been described with reference to the accompanying drawings, one of ordinary skill in the art will understand that various changes and modifications may be made therein. For example, the relevant results may be achieved even when the described technologies are performed in a different order than the described methods, and/or even when the described elements such as systems, structures, devices, and circuits are coupled or combined in a different form than the described methods or are replaced or substituted by other elements or equivalents.

Therefore, other implementations, other embodiments, and equivalents to the claims are also within the scope of the following claims.

According to one of the above-described devices for achieving the problem, it is possible to provide a medical data providing device and a medical data providing method.

In addition, according to one of the above-described devices for achieving the problem, it is possible to reduce the time, power, and resources required for analysis by determining a section in which analysis is unnecessary from a large amount biosignal data measured for a long time.

In addition, according to one of the above-described devices for achieving the problem, it is possible to save a storage space of a biosignal by not storing data of an analysis unnecessary section.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A medical data providing device comprising:
a memory, a communication unit, and a processor,
wherein the processor is configured to
read a biosignal stored in the memory, divide the biosignal at certain time intervals,
convert a signal segment into a frequency domain,
determine whether the signal segment is an analysis necessary section or an analysis unnecessary section based on a frequency component of the signal segment,
further analyze the signal segment of the analysis necessary section to generate data including an analysis result, and
transmit data including the analysis result to a certain device.

2. The medical data providing device of claim 1, wherein
the processor is further configured to
convert the signal segment into frequency-domain data, extract one or more frequency values for one or more peaks included in the signal segment, and set the signal segment as an analysis unnecessary section when there are divisor or multiple frequencies of a largest peak frequency in the first signal segment..

3. The medical data providing device of claim 1, wherein
the processor is further configured to
convert the first signal segment into frequency-domain data, calculate one or more magnitudes of one or more peaks included in the signal segment, set the frequency value of the point of the peak of the highest value as a first frequency, determine whether a plurality of peaks having one or more frequencies of a multipul relation of the first frequency exist in the first signal segment, and the first signal segment is set as an analysis unnecessary section when there are a plurality of peaks in the first signal segment.

4. The medical data providing device of claim 1, wherein
the processor is further configured to
set the signal segment as an analysis unnecessary section when the magnitude of data of one or more first peaks included in the signal segment is less than a preset reference peak value.

5. The medical data providing device of claim 1, wherein
the processor is further configured to
set the signal segment as an analysis unnecessary section when a frequency magnitude of one or more second peaks included in the signal segment exceeds a preset reference frequency value.

6. The medical data providing device of claim 1, wherein
the processor is further configured to
perform a Fourier transform or a fast Fourier transform with a certain sample frequency of the biosignal.

7. The medical data providing device of claim 1, wherein
the processor is further configured to
perform a Fourier transform with the number of samples corresponding to preset time intervals of the biosignal.

8. The medical data providing device of claim 1, wherein
the processor is further configured to
preprocess the signal segment before converting a signal segment of the biosignal into the frequency domain.

9. The medical data providing device of claim 8, wherein
the processor is further configured to
preprocess the signal segment of the biosignal using a zero padding method or a data interpolation method.

10. The medical data providing device of any one of claims 1, 5, and 6, wherein
the processor is further configured to
determine whether the first signal segment is an analysis unnecessary section or an analysis necessary section using an analysis model generated by machine learning.

11. The medical data providing device of claim 1, wherein
the processor is further configured to
determine a length of the time intervals using the analysis model.

12. The medical data providing device of claim 1, wherein
the processor is further configured to
determine the reference peak value or the reference frequency value using the analysis model.

13. A medical data providing method comprising:
reading a biosignal and dividing the biosignal at certain time intervals by a medical data providing device;
converting, by the medical data providing device, a signal segment into a frequency domain;
determining, by the medical data providing device, whether the signal segment is an analysis necessary section or an analysis unnecessary section based on a frequency component of the signal segment; and
generating, by the medical data providing device, data including an analysis result by further analyzing the signal segment of the analysis necessary section and transmitting the data including the analysis result to a certain device.

14. A computer-readable medium storing computer-executable program instructions that, when executed by a processor, cause the processor to perform operations including,
reading a biosignal and dividing the biosignal at certain time intervals;
converting a signal segment into the frequency domain;
determining whether the signal segment is an analysis necessary section or an analysis unnecessary section based on a frequency component of the signal segment; and
generating data including an analysis result by further analyzing the signal segment of the analysis necessary section and transmitting the data including the analysis result to a certain device.
